Europäisches Patentamt

**(19)** European Patent Office

Office européen des brevets

**(11)** Publication number : **0 402 029 B1**

**(12)** EUROPEAN PATENT SPECIFICATION

**(45)** Date of publication of patent specification : **12.01.94 Bulletin 94/02**

**(51)** Int. Cl.⁵ : **A61K 39/42**

**(21)** Application number : **90305844.4**

**(22)** Date of filing : **30.05.90**

**(54)** Monoclonal antibodies for post exposure treatment of rabies.

**(30)** Priority : **08.06.89 US 365143**

**(43)** Date of publication of application : **12.12.90 Bulletin 90/50**

**(45)** Publication of the grant of the patent : **12.01.94 Bulletin 94/02**

**(84)** Designated Contracting States : **AT BE CH DE DK ES FR GB GR IT LI LU NL SE**

**(56)** References cited :
WO-A-87/00179
CHEMICAL ABSTRACTS, vol. 112, no. 7, 12 February 1990, Columbus, OH (US); C.L.SCHUMACHER et al., no. 53398y
CHEMICAL ABSTRACTS, vol. 103, no. 21,Columbus, OH (US); M.LAFON et al., no. 176733a
CHEMICAL ABSTRACTS, vol. 93, no. 7, Columbus, OH (US); A.FLAMAND et al., no. 68315g

**(56)** References cited :
REVIEWS OF INFECTIONS DISEASES, vol. 10, suppl. 4, 1988; pp. S785-S798
VIROLOGY, vol. 161, 1987; pp. 29-36
JOURNAL GEN. VIROL., vol. 66, 1985; pp. 2125-2133
JOURNAL GEN. VIROL., vol. 48, 1980; pp. 97-104, and 105-109

**(73)** Proprietor : **THE WISTAR INSTITUTE Thirty-Sixth Street at Spruce Philadelphia Pennsylvania 19104-4268 (US)**

**(72)** Inventor : **Dietzschold, Bernhard 3034 Goshen Road Newton Square, PA 19073 (US)**
Inventor : **Rupprecht, Charles E. 214 Wedgewood Drive Turnersville, NJ 08012 (US)**

**(74)** Representative : **Dean, John Paul et al Withers & Rogers 4 Dyer's Buildings Holborn London EC1N 2JT (GB)**

## Description

Technical Field of the Invention

The invention relates to the field of anti-viral therapy. More particularly it relates to treatment after rabies virus has been transmitted to a new host organism.

## BACKGROUND OF THE INVENTION

Rabies remains a significant human disease throughout the developing world: for example, approximately 500,000 persons (Steele, H.H. 1988, "Rabies in the Americas and Remarks on Global Aspects," Rev. Infect. Dis., 10 (Suppl. 4):585) undergo anti-rabies treatment in India and some 40,000-50,000 people are reported to die of rabies each year (Baer, G.M. 1988, "Research Towards Rabies Prevention: Overview," Rev. Infect. Dis., 10 (Suppl. 43:576).

Effective post-exposure treatment of rabies as recommended by the World Health Organization (WHO) includes the prompt use of human or equine anti-rabies immunoglobulins (HRIG or ERIG, respectively) together with the administration of vaccine. Animal experiments have demonstrated that treatment with vaccine alone does not prevent lethal rabies virus infection in post-exposure situations (Koprowski, H., and J. Van Der Scheer, 1950, "Use of Hyperimmune Anti-rabies Serum Concentrates in Experimental Rabies," Am. J. Med., 8:412: Sikes, R.K., W.F. Clearly, H. Koprowski, T.J. Wiktor, and M. Kaplan, 1971, "Effective Protection of Monkeys Against Death by Street Virus by Post-Exposure Administration of Tissue Culture Rabies Vaccine, Bull. WHO 45:1). Anti-rabies antibodies appear to be an essential component in the treatment of rabies: however, the precise role of such antibodies in post-exposure treatment is unclear.

Possible mechanisms of action of rabies virus neutralizing antibodies (VNA), solely directed against the rabies virus glycoprotein (G protein), include neutralization of extra-cellular rabies virus, complement-mediated lysis of rabies virus-infected cells and antibody-dependent cellular cytotoxicity (Davis, D.R., and H. Metzger 1983, "Structural Basis of Antibody Function," Annu. Rev. Immunol. 1:87). Aside from these effector functions of VNA, other antibodies, including those which are directed against internal nucleocapsid protein (RNP), are capable of regulating the immune response of T lymphocytes (Celis, E., Wiktor, T.J., Dietzschold, B. and Koprowski, H. 1985, "Amplification of Rabies Virus-Induced Stimulation of Human T-cell Lines and Clones by Antigen-Specilfic Antibody," J. Virol., 56, 426-433). It was recently demonstrated that antibodies which recognize the G-protein or RNP proteins play an important role in potentiating the antigen-induced proliferative response and production of gamma interferon (IFN-gamma) of rabies virus-specific T-lymphocytes and IFN-gamma was shown to play a role in the protection against lethal rabies virus infection (Dietzschold, B., M. Gore, H. Ertl, E. Celis, J. Otvos, Jr., and H. Koprowski, 1989, "Analysis of Protective Immune Mechanisms Induced by Rabies Nucleoprotein," In: Genetics and Pathogenicity of Negative Strand Viruses, B. W.J. Mahy and D. Kalakowsky, ed., Elsevier, New York, 295).

Because of the potential risk of allergic reactions connected with ERIG use, only HRIG is ideally recommended for post-exposure treatment of humans. However, due to the high cost of HRIG and the high incidence of human rabies exposures, there is often insufficient HRIG available to initiate the full recommended post-exposure treatment. Limited access to HRIG is probably a major contributing factor in the increasing number of post-exposure treatment failures (Anonymous (Editorial) 1988, "Rabies Vaccine Failures," Lancet, April 23, 912). Additionally, safety aspects of human immune serum treatment must be considered, due to the potential risk of hepatitis B virus and human immunodeficiency virus contamination. For these reasons, there is a need in the art for a safe and readily available substitute for HRIG, which can be used in conjunction with vaccine in the post-exposure treatment of rabies.

## SUMMARY OF THE INVENTION

It is an object of the invention to provide a composition for post-exposure treatment of rabies infection.

It is another object of the invention to provide a composition comprising a mixture of monoclonal antibodies.

These and other objects of the invention are provided by one or more of the embodiments described below. In one embodiment a composition is provided comprising a mixture of monoclonal antibodies of the IgG isotype, at least two of said antibodies immunoreactive with two different epitopes on the G protein of rabies virus, at least one of said antibodies immunoreactive with the N protein of rabies virus, at least one of said antibodies immunoreactive with all street and fixed rabies viruses, at least one of said antibodies immunoreactive with Duvenhage virus, at least one of said antibodies immunoreactive with Mokola virus.

This invention provides the art with a safe and reliable substitute for HRIG to treat rabies infection after exposure.

## DETAILED DESCRIPTION OF THE INVENTION

It is a discovery of the present invention that a cocktail of monoclonal antibodies can provide protection to mammals which have been infected with rabies virus. The cocktail may contain human, mouse or other species monoclonal antibodies. Because of the generally higher specific activity of monoclonal antibody mixtures compared to whole, hyperimmune antisera, a smaller amount of antibody protein must be administered. This provides benefits for local wound treatment, where only small volumes can be inoculated at the bite site. In addition, the risk of anaphylactic reactions and hypersensitization are reduced relative to whole heterologous hyperimmune serum because of the small amounts which must be administered.

The cocktail or mixture of the present invention is composed of monoclonal antibodies. The species source of the antibodies is not critical, although use of homologous antibodies may reduce the risk of anaphylactic shock and hypersensitization. Methods of making monoclonal antibodies are well known in the art. See, e.g., Wiktor, et al., Proc. Natl. Acad. Sci., USA, vol. 75, pp. 3938-3942, 1978. Preferred antibodies for use in the present invention are mouse antibodies.

The composition of the present invention employs antibodies of the IgG isotype. IgM antibodies are thought not to be suitable. Methods of determining the isotype of an antibody are well known in the art. For example, commercial antibodies are available for determining isotype. The immunological specificity of the antibodies of the present invention is preferably as broad as possible in terms of the number of types of viruses with which they will react. It is desirable that at least one of the antibodies be immunoreactive with the rabies-related Duvenhage virus and at least one of the antibodies be immunoreactive with the rabies-related Mokola virus. It is also desirable that a broad range of street and fixed rabies viruses be represented in the immunoreactivity spectrum of the antibodies of the cocktail. Preferably all street and fixed rabies viruses will be represented. Fixed viruses are modified by serial passage in laboratory animals. Street viruses are wild-type viruses.

The epitopic specificity of the antibodies of the mixture are desirably as diverse as possible. Desirably, some antibodies will be immunoreactive with the G protein (glycoprotein) of rabies virus while some antibodies will be immunoreactive with the N protein (nucleoprotein) of rabies virus. At a minimum, two different anti-G antibodies are desirably used, each directed to a different epitope of the G protein. In a preferred embodiment, antigenic sites I, IIc, and IIIb of the G protein are each represented in the immunospecifity of the mixture. Particular antibodies which may be used to react with these epitopic sites are MAb 509-6, (Flamand, et al., J. Gen. Virol., vol. 48, p. 105, 1980) MAb1112-1, (Dietzschold, Review of Infections Disease, vol. 10, p. 785 supp. 1988) and MAb 523-11 (Flamand, et al., J. Gen. Virol, vol. 48, p. 105, 1980,) respectively. If more than one anti-N antibody are used, then the N protein epitopes with which they react should also be diverse. For example, epitopic sites II and III can be represented by MAb 801-1 and MAb 502-2 (LaFon et al., J. Gen. Virol, vol. 66, p. 2125, 1985, and Flamand, et al., J. Gen. Virol, vol. 48, p. 97, 1980, respectively.)

A protective dose of antibody mixture is generally administered all at one time. Repeated dosing is not required. The dose is generally between about 50 and about 200 I.U./Kg of body weight. It is believed that higher doses are desirable when the amount of time between exposure and therapy is long. When therapy is given within a few hours, lower doses be used. However, when a few days have elapsed since exposure, higher doses are indicated.

As in therapeutic regimens employing ERIG and HRIG, in most cases it will be desirable to administer a rabies vaccine along with the mixture of antibodies of the present invention. Any conventional rabies vaccine can be used, as is known in the art. Generally these are rabies viruses which have been attenuated, although genetically engineered rabies proteins or immunogenic synthetic peptides may also be used.

The following examples are provided as illustrative of particular embodiments only. They are not intended to limit the scope of the invention described above and by the claims appended below.

## Example 1

This example describes the properties of the antibodies selected from the protective cocktail of the present invention.

Five immune MAbs were selected form a panel of 76 G protein and RNP-specific MAbs (Table 1). The criteria for the selection were based on isotype, antigen and epitope specificity, virus strain specificity, affinity and virus neutralizing activity. As shown in Table 1, three of the MAbs were specfific for the G protein; the other two non-neutralizing MAbs were specific for RNP. Each of the three G protein-specific antibodies recognizes a different antigenic site on the G protein; the epitope recognized by MAb 509-6 is located within site I, the epitope for MAb 1112-1 is located in site IIc and the epitope of MAb 523-11 is located in the IIIb (Dietzschold, B., Rupprecht, C.E., Tollis, M. Lafon, M., Mattei, J., Wiktor, T.J. And Koprowski, H., 1988 "Antigenic diversity of the glycorprotein and nucleoprotein of rabies and rabies-related viruses: Implications for Epidemiology and Control of Rabies," Review of Infectious Diseases, vol. 10, (Suppl. 4), 5785-5798.) All three G protein-specific MAbs are able to neutralize in vitro all fixed rabies virus strains and most of the field rabies virus isolates (Dietzschold, B., Rupprecht, C.E., Tollis, M. Lafon, M., Mattei, J., Wiktor, T.J. and Koprowski, H., 1988, "Antigenic diversity of the glycoprotein and nucleoprotein of rabies and rabies-related viruses: Implications for Epidemiology and Control of Rabies," Review of Infectious Diseases, vol. 10, (Suppl. 4), 5785-5798.) In addition, MAb 1112-1 neutralized the rabies related strains Duvenhage 1-6 and MAb 523-11 neutralized the Mokala 3 virus (Dietzschold, B., Rupprecht, C.E., Tollis, M. Lafon, M., Mattei, J., Wiktor, T.J. and Koprowski, H., 1988. "Antigenic diversity of the glycoprotein and nucleoprotein of rabies and rabies-related viruses: Implications for Epidemiology and Control of Rabies," Review of Infectious Diseases, vol. 10, (Suppl. 4), 5785-5798.)

It can be seen from Table 1 that the three G protein-specific MAbs differ largely in their virus neutralizing activity. Virus neuturalization was determined by a modification of the rapid fluorescent focus inhibition test, as previously described (Wiktor, T.J., et al., 1984, "Antigenic Analysis of Rabies and Mokola Virus from Zimbabwe Using Monoclonal Antibodies," Dev. Biol. Stand. 57:199.) A reduction of viral titer greater than 100 infective units was considered as positive virus neutralization. While the specific virus neutralizing activities of MAb 509-6 and 523-11 are very high (1667 I.U./mg and 8242 I.U./mg) the virus neutralizing activity in vitro of MAb 1112-1 is low (62 I.U./ug). However, the specific virus neutralizing activity of all three MAbs are definitively superior to that of HRIG which only 1.13 I.U./mg protein.

The RNP specific MAbs 802-1 and 502-2 recognize epitopes located in site II and site III of the N protein, respectively (Dietzschold, B., et al., 1988, "Antigenic Diversity of the Glycoprotein and Nucleoprotein of Rabies and Rabies-Related Viruses: Implications for Epidemiology and Control of Rabies," Review of Infectious Disease, Vol. 10, (Suppl. 4) 5785-5798.) MAb 502-2 reacts with all known rabies and rabies related virus strains (Dietzschold, B., et al., 1988, "Antigenic Diversity of the Glycoprotein and Nucleoprotein of Rabies and Rabies-Related Viruses: Implications for Epidemiology and Control of Rabies," Review of Infectious Disease, Vol. 10, (Suppl. 4) 5785-5798.)

All five MAbs were used in a MAb cocktail which represents a mixture of 112.5 I.U./ml MAb 509-6, 10 I.U./ml of MAb 1112-1, 800 I.U./ml of MAb 523-11, 0.04 mg/ml MAb 801-1 and 0.6 mg/ml MAb 502-2. The specific virus neutralizing activity of the cocktail was 1376 I.U./mg protein.

## Example 2

This example demonstrates the prophylactic efficacy of three individual MAbs directed against the G protein of rabies. In addition, the involvement of the Fc portion of antibodies in the prophylactic response is shown.

To determine prophylactic efficacy, various dilutions of the individual MAbs were inoculated intramuscularly (i.m.) into ICR mice, and 24 hrs. later the animals were challenged intracerebrally with a lethal dose ($5 \times 10^4$ of mouse $LD_{50}$) of the CVS 11 strain of rabies virus. While pretreatment of mice with RNP specific MAbs had no effect on survival (data not shown), administration of the G protein specific MAbs 24 hrs. before challenge effectively prevented a lethal rabies virus infection (Table 2). Two I.U. of MAb 509-6 and 2.36 I.U. of MAb 523-11 were necessary to protect 50% of the mice. Although the in vitro virus neutralizing activity of MAb 1112-1 was low, its protective activity in vivo was extremely high. As little as 0.03 I.U. of this MAb was sufficient to prevent a lethal rabies virus infection in 50% of the animals. The explanation for the discrepancy between in vivo and in vitro activity of MAb 1112-1 is not clear. It is likely that in contrast to an in vitro neutralization, the mechanism of MAbs in vivo is very complex and probably not only based on the neutralization of extracellular virus.

To demonstrate that the constant region of the antibody which contains the Fc fragment plays an important role in the in vivo protection from rabies, mice were treated with F(ab')$_2$ fragments (lacking the Fc) of MAb 523-11. Table 2 shows that the $ED_{50}$ of F(ab')$_2$ fragments is 34.0 I.U. compared to a $ED_{50}$ of 2.36 of the intact MAb 523-11. Thus whole antibody is much more effective than F(ab')2 fragments.

## Example 3

This example demonstrates that the amount of time that elapses between MAb treatment and virus challenge has a strong influence on the protective activity of the antibody.

Mice were challenged intramuscularly with $5 \times 10^6$ MIC $LD_{50}$ (lethal dose for 50% of the mice) of CVS 24 [obtained from Wistar Institute collection.] At various times before and after challenge with virus, different doses of the monoclonal antibody cocktail were administered.

It is shown in Table 3 that the $ED_{50}$ of the MAb cocktail is 0.8 when administered 24 hrs before challenge, but increases to 2.36 when the MAbs are given 2 hrs before challenge. Moreover when the MAb cocktail is injected 2 hrs after challenge 42.8 I.U. are necessary to protect 50% of the mice.

## Example 4

This example demonstrates that the site at which the viral challenge is administered has a dramatic effect on the measured efficacy of the monoclonal antibody cocktail.

Two challenge groups of mice were treated with dilutions of the MAb cocktail and 24 hrs later challenged intracerebrally (i.c.) or intrafootpad (i.f.) with a lethal dose ($5 \times 10^4$ MIC $LD_{50}$) of rabies virus (CVS11). Even when 200 I.U. were administered 24 hrs before challenge only one out of seven mice survived an i.c. challenge (see Table 4). The ineffectiveness of MAb treatment against an i.c. challenge is puzzling since mice that received 200 I.U. of MAbs had a circulating VNA titer of 3992 (GMT) 24 hrs after injection but were not protected. In contrast, most mice immunized with beta-propiolactone (BPL) inactivated rabies virus had a circulating VNA titer of only 767 (GMT) and survived the i.c. challenge (Table 4).

When the challenge infection was given into the foot pad (data not shown) 4 I.U of MAb cocktail administered 24 hours before challenge were necessary, to protect 50% of mice against an i.f. challenge. Thus the $ED_{50}$ of MAbs against an i.f. challenge is 5 times higher than the $ED_{50}$ of the same MAbs against an i.m. challenge (c.f. Table 3).

## Example 5

This example demonstrates that the mouse monoclonal antibody mixture of the present invention is effective in preventing mortality even when administered after exposure to rabies virus.

Because most pathogenic rabies virus strains have a very short incubation time in mice (onset of clinical signs is 5-6 days after infection) it is difficult to perform post-exposure treatment experiments in mice. Since the incubation period in hamsters is much longer (10-14 days) these animals species are a more appropriate model for post-exposure treatment experiments.

To assess the efficacy of MAbs in a post-exposure situation, groups of 5 hamsters were inoculated i.m. with NYC strain rabies street virus. Two, three and thirty-six hrs. post-infection, five hamsters were treated i.m. with 1550 or 150 I.U./kg of the MAb cocktail. Table 5 shows that all hamsters which received the antibody two or three hours after challenge survived, while 80% of the untreated control animals succumbed to rabies infection. Treatment with the MAb cocktail 36 hrs. after infection resulted in 80% survival.

## Experimental Methods Employed

**Virus strains.** The CVS-11, ERA and PM strains of fixed rabies virus as well as street rabies virus isolates and the rabies-related Duvenhage-6 (DUV-6) and Mokola (MOK) viruses were propagated in BHK-21 cell culture monolayers as previously described (Wiktor, T.J., 1977, "Induction and Biological Properties of Defective Interfering Particles of Rabies Virus," J. Virol. 21:626.)

**Monoclonal antibodies (MAbs).** Hybridomas that secrete MAbs specific for rabies glycoprotein (G protein) and nucleocapsid protein (RNP) were produced by the fusion of P3 X 63 Ag8 or 654 myeloma cells with splenocytes of BALB/c mice immunized with several strains of rabies virus (Wiktor, T.J., 1978, "Monoclonal Antibodies Against Rabies Virus Produced by Somatic Cell Hybridization Detection of Antigenic Variants," Proc. Natl. Acad. Sci. U.S.A., 75:3938-3942.)

**Purification of MAbs.** MAbs were precipitated from ascites fluid with $(NH_4) SO_4$ as described (Hughes, E.N., et al., 1982, "Murine Cell Surface Glycoproteins: Identification, Purification and Characterization of a Major Component of 110,000 Daltons by Use of a Monoclonal Antibody," J. Biol. Chem., 257:3970-3977.) The precipitate was dissolved in PBS dialized against 25 mM MES [2-(N-Morpholino) ethanesulfonic acid) pH 5.5 and subjected to HPLC ion exchange chromatography employing a Baker Bound Abx 15 micron Prep column (Baker Chemical Company). Chromatography conditions were similar to previously described (Nau, D.R., 1986, "Rapid Antibody Purification: Bakerbond Abx, J.T. Baker Product Information Bulletin No. 8506, J.T. Baker Chemical Company). Solvent A was 25 mM MES pH 5.6, solvent B was 1M sodium acetate pH 7.0. A gradient of 0.70% B was run for 60 minutes at a flow rate of 3 ml/min at ambient temperature. The elution of proteins was monitored by UV absorbance at 280 nm. Peak fractions containing the MAb were pooled, dialyzed against PBS and concentrated by ultrafiltration.

**Preparation of F(ab')$_2$.** F(ab')$_2$ fragments were prepared from purified MAbs as described (Chang, T.W., et al., 1982, "Cellular Origin and Interactions Involved in Gamma-Interferon Production Induced by OKT3 Monoclonal Antibody," J. Immunol. 128:585.) Fifteen mg of MAb in 10 ml 0.1 mM sodium acetate buffer, pH 4.5, were mixed with 0.15 mg of pepsin (Millpore Corporation, U.S.A.) and incubated at 37°C for 16 hours. The digest was dialized against PBS and subjected to chromatography on a protein A sepharose CL-4 B column. No residual intact IgG was detectable in the $F(ab')_2$ preparation when analyzed by SDS-polyacrylamide gel electrophorsis.

**In vivo protection experiments in mice.** Six week old female ICR mice (Harlan Sprague-Dawley) in groups of seven or ten were injected intramuscularly (i.m.) into the right gastronemius muscle with 0.1 ml of one of five serial dilutions (100 I.U. - 0.16 I.U.) of mouse MAbs. Twenty-four hours after MAb treatment all mice were challenged i.m. in the left gastronemius muscle with 10 MIC $LD_{50}$ of CVS-24 virus. Animals were observed for three weeks and deaths were reported daily. The effective dose ($ED_{50}$) of MAbs was calculated as described (Habel, K., 1966, "Procedure for Determining 50% Endpoints of Habel-type Potency Test in Mice. In: Laboratory Techniques in Rabies, Second Ed., World Health Organization, Monograph Series No. 23, 142-143.)

**Post-exposure treatment of hamsters with mouse MAbs.** Syrian Hamsters at 4-6 weeks of age were used for all experiments. Challenge virus consisted of a 20% salivary gland suspension of a street rabies virus isolate of fox origin; dilutions were made in distilled water containing horse serum. For each trial, hamsters were divided into groups of five and received 100ul of either PBS (controls) or monoclonal antibodies (MAbs), as follows:

## Trial 1

Hamsters received $10^{5.1}$ MIC $LD_{50}$/ml of rabies virus and treated with monoclonal antibody three hrs. or thirty-six hrs. later with murine MAbs (15351U/ml).

## Trial 2

Hamsters received $10^{4.6}$ MIC $LD_{50}$/ml of rabies virus and were treated two hrs. later with murine MAbs (155 IU/ml).

Animals were examined daily for definitive clinical signs of rabies infection and were euthanized when in extremis by $CO_2$ intoxication. Survivors were followed for at least 30 days following rabies virus inoculation at which time they were similarly euthanized.

# TABLE 1

## MOUSE MONOCLONAL ANTIBODIES USED FOR PASSIVE PROTECTION AGAINST RABIES

| MAb # | ISOTYPE | ANTIGEN SPECIFICITY | SPECIFIC VIRUS NEUTRALIZING ACTIVITY (I.U./mg.) | VIRUS STRAIN SPECIFICITY |
|---|---|---|---|---|
| 509-6 | $\gamma$ 2a | G | 1667 | Neutralization of all fixed and street rabies virus strains except some virus isolates from bats. |
| 1112-1 | $\gamma$ 1 | G | 61 | Neutralization of all fixed and street rabies virus strains and the rabies-related Duvenhage strains. |
| 523-11 | $\gamma$ 2a | G | 8242 | Neutralization of all fixed and street rabies virus strains except some street virus isolates from bats and foxes. |
| 801-1 | $\gamma$ 2b | N | 0 | Binding to all fixed and street rabies virus strains and some rabies-related viruses. |
| 502-2 | $\gamma$ 2a | N | 0 | Binding to all rabies and rabies-related viruses. |
| MAb Cocktail* | | G,N | 1376 | N.D. |

* The MAb cocktail represents a mixture of 112.5 I.U./ml MAb 509-6, 10 I.U./ml of MAb 1112-1, 800 I.U./ml MAb 523-11, 0.04 mg/ml MAb 801-1, and 0.6 mg/ml MAb 502-2.

EP 0 402 029 B1

Table 2.

# PASSIVE PROTECTION OF ICR MICE WITH MAb COCKTAIL

### M O R T A L I T Y

| ANTIBODY DOSE (IU) | MAb 1112-1 24 HRS BEFORE I.M. CHALLENGE | MAb 509-6 24 HRS BEFORE I.M. CHALLENGE | MAb 523-11 24 HRS BEFORE I.M. CHALLENGE | F(ab)$_2$ 523-11 24 HRS BEFORE I.M. CHALLENGE |
|---|---|---|---|---|
| 100 | -- | -- | 0/10 | 2/11 |
| 20 | -- | 1/10 | 2/11 | 7/10 |
| 4 | 0/10 | 3/10 | 8/10 | 10/10 |
| 0.8 | 1/10 | 7/10 | 10/10 | 10/10 |
| 0.16 | 0/10 | -- | -- | -- |
| 0.032 | 2/10 | -- | -- | -- |
| ED$_{50}$ (I.U.) | <0.03 | 2.00 | 2.36 | 34.00 |

Mice were challenged i.c. with $5 \times 10^4$ MIC LD$_{50}$ of CVS 11

EP 0 402 029 B1

**Table 3**

EP 0 402 029 B1

## PASSIVE PROTECTION OF ICR MICE WITH MAb COCKTAIL

### M O R T A L I T Y

| ANTIBODY DOSE (IU) | Mab COCKTAIL 24 HRS BEFORE CHALLENGE | MAb COCKTAIL 2 HRS BEFORE CHALLENGE | MAb COCKTAIL 2 HRS AFTER CHALLENGE |
|---|---|---|---|
| 100 | 0/10 | 0/7 | 3/7 |
| 20 | 0/10 | 1/10 | 8/10 |
| 4 | 0/10 | 4/10 | 8/10 |
| 0.8 | 5/10 | 7/10 | 9/10 |
| 0.16 | 10/10 | -- | -- |
| $ED_{50}$ (I.U.) | 0.80 | 2.36 | 42.80 |

Mice were challenged i.m. with $5 \times 10^6$ MIC $LD_{50}$ of CVS 24

## TABLE 4

VNA titers and mortality rates after MAB treatment or immunization with BPL-inactivated ERA virus.

| MAB treatment* (200 I.U./mouse) | | Immunization with BPL ERA * * (2ug/mouse) | |
|---|---|---|---|
| *** VNA titer (GMT, n=7) | Mortality | *** VNA titer (GMT, n=7) | Mortality |
| 3992 (2430-7290) | 6/7 | 767 (90-3125) | 1/7 |

*Mice were treated with 200 I.U. of MAB cocktail and 24 hr. later challenged i.c. with $5 \times 10^4$ MIC $LD_{50}$ of CVS 11

**Mice were immunized with 2 ug of BPL inactivated ERA virus on day 0 an d7 and challenged on day 14 i.c. with $5 \times 10^4$ MIC $LD_{50}$ of CVS 11.

*** Mice were bled 2 hr before challange.

## POST-EXPOSURE RABIES TREATMENT OF SYRIAN HAMSTERS
## WITH MOUSE MONOCLONAL ANTIBODIES*

TABLE 5

| ANTIBODY DOSE (IU/Kg) | TIME OF TREATMENT AFTER VIRUS CHALLENGE(HRS) | CHALLENGE VIRUS DILUTION** | MORBIDITY |
|---|---|---|---|
| 1550 | 3 | 1:200 | 0/5 |
| 1550 | 36 | 1:200 | 1/5 |
| 150 | 2 | 1:1000 | 0/5 |
| 0 | - | 1:200 | 4/5 |
| 0 | - | 1:1000 | 4/5 |

\* Antibodies were administered intramuscularly.

\*\* Hamsters were challenged intramuscularly with a suspension of dog salivary gland infected with the NYC strain of rabies virus.

EP 0 402 029 B1

EP 0 402 029 B1

**Claims**

**Claims for the following Contracting States : AT, BE, CH, DE, GB, FR, IT, LU, NL, SE, DK**

1.  A composition for post-exposure treatment of rabies infection comprising a mixture of monoclonal antibodies of the IgG isotype, at least two of said antibodies being immunoreactive with two different epitopes on the G protein of rabies virus, at least one of said antibodies being immunoreactive with the N protein of rabies virus, at least one of said antibodies being immunoreactive with all street and fixed rabies viruses, at least one of said antibodies being immunoreactive with Duvenhage virus, at least one of said antibodies being immunoreactive with Mokola virus.

2.  The composition of claim 1 wherein the monoclonal antibodies are mouse antibodies.

3.  The composition according to claim 1 comprising antibodies recognising the following sites: sites I, IIc, and IIIb of the G protein of rabies virus and sites II and III of the N protein of rabies virus.

4.  The composition of claim 2 wherein the monoclonal antibodies are those designated: 509-6, 1112-1, 523-11, 801-1 and 502-2.

5.  The composition of claim 4 wherein the relative proportions of the antibodies are as follows: 112.5 I.U. /ml MAb 509-6, 10 I.U./ml MAb 1112-1, 800 I.U./ml of MAb 523-11, 0.04 mg/ml MAb 801-1 and 0.6 mg/ml MAb 502-2.

6.  The composition of any preceding claim for intramuscular administration.

7.  A composition according to any preceding claim which comprises a protective dose of between about 50 and about 2000 I.U./kg of body weight.

**Claims for the following Contracting States : ES, GR**

1.  A method of preparing a composition for post-exposure treatment of rabies infection the method comprising preparing a mixture of monoclonal antibodies of the IgG isotype, at least two of said antibodies being immunoreactive with two different epitopes on the G protein of rabies virus, at least one of said antibodies being immunoreactive with the N protein of rabies virus, at least one of said antibodies being immunoreactive with all street and fixed rabies viruses, at least one of said antibodies being immunoreactive with Duvenhage virus, at least one of said antibodies being immunoreactive with Mokola virus.

2.  A method according to claim 1 wherein the monoclonal antibodies are mouse antibodies.

3.  A method according to claim 1 in which the antibodies recognise the following sites: sites I, IIc, and IIIb of the G protein of rabies virus and sites II and III of the N protein of rabies virus.

4.  A method according to claim 2 wherein the monoclonal antibodies are those designated: 509-6, 1112-1, 523-11, 801-1 and 502-2.

5.  A method according claim 4 wherein the relative proportions of the antibodies are as follows: 112.5 I.U. /ml MAb 509-6, 10 I.U./ml MAb 1112-1, 800 I.U./ml of MAb 523-11, 0.04 mg/ml MAb 801-1 and 0.6 mg/ml MAb 502-2.

6.  A method according to any preceding claim in which the composition is for intramuscular administration.

7.  A method according to any preceding claim in which the composition comprises a protective dose of between about 50 and about 2000 I.U./kg of body weight.

**Patentansprüche**

**Patentansprüche für folgende Vertragsstaaten : AT, BE, CH, DE, DK, GB, FR, IT, LU, NL, SE**

1.  Zusammensetzung zur Behandlung von Rabies-Infektionen (Tollwut-Infektionen) nach einem Kontakt mit dem Tollwutvirus, die ein Gemisch aus monoklonalen Antikörpern des IgG-Isotyps umfaßt, wobei wenigstens zwei der Antikörper mit zwei verschiedenen Epitopen auf dem G Protein des Tollwutvirus immunoreaktiv sind, wobei wenigstens einer der Antikörper mit dem N Protein des Tollwutvirus immunoreaktiv ist, wobei wenigstens einer der Antikörper mit allen Straßen- (street) und stabilen (fixed) Tollwutviren immunoreaktiv ist, wobei wenigstens einer der Antikörper mit dem Duvenhage-Virus immunoreaktiv ist, und wobei wenigstens einer der Antikörper mit dem Mokola-Virus immunoreaktiv ist.

2. Die Zusammensetzung nach Anspruch 1, wobei die monoklonalen Antikörper Mäuse-Antikörper sind.

3. Die Zusammensetzung nach Anspruch 1, die Antikörper umfaßt, die die folgenden Stellen (sites) erkennen: die Stellen I, IIc und IIIb des G Proteins des Tollwutvirus und die Stellen II und III des N Proteins des Tollwutvirus.

4. Die Zusammensetzung nach Anspruch 2, wobei die monoklonalen Antikörper diejenigen Antikörper sind, die wie folgt bezeichnet werden: 509-6, 1112-1, 523-11, 801-1 und 502-2.

5. Die Zusammensetzung nach Anspruch 4, wobei die relativen Verhältnisse der Antikörper wie folgt sind: 112,5 I.E.- /ml MAb 509-6, 10 I.E./ml MAb 1112-1, 800 I.E./ml MAb 523-11, 0,04 mg/ml MAb 801-1 und 0,6 mg/ml MAb 502-2.

6. Die Zusammensetzung nach einem der vorhergehenden Ansprüche für die intramuskuläre Verabreichung.

7. Die Zusammensetzung nach einem der vorhergehenden An sprüche, die eine Schutzdosis von zwischen etwa 50 und etwa 2000 I.E./kg Körpergewicht enthält.

**Patentansprüche für folgende Vertragsstaaten : ES, GR**

1. Verfahren zur Herstellung einer Zusammensetzung zur Behandlung von Rabies-Infektionen (Tollwut-Infektionen) nach einem Kontakt mit dem Tollwutvirus, wobei das Verfahren die Herstellung eines Gemisches aus monoklonalen Antikörpern des IgG-Isotyps umfaßt, wobei wenigstens zwei der Antikörper mit zwei verschiedenen Epitopen auf dem G Protein des Tollwutvirus immunoreaktiv sind, wobei wenigßtens einer der Antikörper mit dem N Protein des Tollwutvirus immunoreaktiv ist, wobei wenigstens einer der Antikörper mit allen Straßen- (street) und stabilen (fixed) Tollwutviren immunoreaktiv ist, wobei wenigstens einer der Antikörper mit dem Duvenhage-Virus immunoreaktiv sind, und wobei wenigstens einer der Antikörper mit dem Mokola-Virus immunoreaktiv ist.

2. Das Verfahren nach Anspruch 1, wobei die monoklonalen Antikörper Mäuse-Antikörper sind.

3. Das Verfahren nach Anspruch 1, wobei die Antikörper die folgenden Stellen (sites) erkennen: die Stellen I, IIc und IIIb des G Proteins des Tollwutvirus und die Stellen II und III des N Proteine des Tollwutvirus.

4. Das Verfahren nach Anspruch 2, wobei die monoklonalen Antikörper diejenigen Antikörper sind, die wie folgt bezeichnet werden: 509-6, 1112-1, 523-11, 801-1 und 502-2.

5. Das Verfahren nach Anspruch 4, wobei die relativen Verhältnisse der Antikörper wie folgt sind: 112,5 I.E./ml MAb 509-6, 10 I.E./ml MAb 1112-1, 800 I.E./ml MAb 523-11, 0,04 mg/ml MAb 801-1 und 0,6 mg/ml MAb 502-2.

6. Verfahren nach einem der vorhergehenden Ansprüche, wobei die Zusammensetzung für die intramuskuläre Verabreichung ist.

7. Das Verfahren nach einem der vorhergehenden Anprüche, wobei die Zusammensetzung eine Schutzdosis von zwischen etwa 50 und etwa 2000 I.E./kg Körpergewicht enthält.

**Revendications**

**Revendications pour les Etats contractants suivants : AT, BE, CH, DE, DK, GB, FR, IT, LU, NL, SE**

1. Une composition pour le traitement curatif de la rage comprenant un mélange d'anticorps monoclonaux de l'isotype IgG, deux au moins desdits anticorps étant immunoréactifs par rapport à deux épitopes différents sur la protéine G du virus de la rage, l'un au moins desdits anticorps étant immunoréactif par rapport à la protéine N du virus de la rage, l'un au moins desdits anticorps étant immunoréactif par rapport à tous les virus contagieux ou atténués de la rage, l'un au moins desdits anticorps étant immunoréactif par rapport au virus Duvenhage, l'un au moins desdits anticorps étant immunoréactif par rapport au virus Mokola.

2. La composition de la revendication 1, dans laquelle les anticorps monoclonaux sont des anticorps de souris.

3. La composition de la revendication 1 comprenant des anticorps qui reconnaissent les sites suivants : les sites I, IIc, et IIIb de la protéine G du virus de la rage et les sites II et III de la protéine N du virus de la rage.

4. La composition de la revendication 2, dans laquelle les anticorps monoclonaux sont ceux désignés par les numéraux suivants : 509-6, 1112-1, 523-11, 801-1 et 502-2.

5. La composition de la revendication 4, dans laquelle les proportions relatives des anticorps entre eux sont : 112,5 I.U. /ml MAb 509-6, 10 I.U. /ml MAb 1112-1, 800 I.U. /ml de MAb 523-11, 0,04 mg/ml MAb 801-1 et 0,6 mg/ml MAb 502-2.

6. La composition de l'une quelconque des revendications précédentes, laquelle est destinée à être administrée par voie intramusculaire.

7. La composition selon l'une quelconque des revendications précédentes, laquelle comprend une dose protectrice allant d'environ 50 à environ 2000 I.U./kg de poids corporel.

**Revendications pour les Etats contractants suivants : ES, GR**

1. Une méthode de préparation d'une composition permettant le traitement curatif de la rage, la méthode comprenant la préparation d'un mélange d'anticorps monoclonaux de l'isotype IgG, deux au moins desdits anticorps étant immunoréactifs par rapport à deux épitopes différents sur la protéine G du virus de la rage, l'un au moins desdits anticorps étant immunoréactif par rapport à la protéine N du virus de la rage, l'un au moins desdits anticorps étant immunoréactif par rapport à tous les virus contagieux ou atténués de la rage, l'un au moins desdits anticorps étant immunoréactif par rapport au virus Duvenhage, l'un au moins desdits anticorps étant immunoréactif par rapport au virus Mokola.

2. Une méthode selon la revendication 1, dans laquelle les anticorps monoclonaux sont des anticorps de souris.

3. Une méthode selon la revendication 1, dans laquelle les anticorps reconnaissent les sites suivants : les sites I, IIc, et IIIb de la protéine G du virus de la rage et les sites II et III de la protéine N du virus de la rage.

4. Une méthode selon la revendication 2, dans laquelle les anticorps monoclonaux sont ceux désignés par les numéraux suivants : 509-6, 1112-1, 523-11, 801-1 et 502-2.

5. Une méthode selon la revendication 4, dans laquelle les proportions relatives des anticorps entre eux sont : 112,5 I.U. /ml MAb 509-6, 10 I.U. /ml MAb 1112-1, 800 I.U. /ml de MAb 523-11, 0,04 mg/ml MAb 801-1 et 0,6 mg/ml MAb 502-2.

6. Une méthode selon l'une quelconque des revendications précédentes, dans laquelle la composition est administrée par voie intramusculaire.

7. Une méthode selon l'une quelconque des revendications précédentes, dans laquelle la composition comprend une dose protectrice allant d'environ 50 à environ 2000 I.U./kg de poids corporel.